**Europäisches Patentamt**

**European Patent Office**

**Office européen des brevets**

(19)

(11) Publication number : **0 444 074 B1**

## (12) EUROPEAN PATENT SPECIFICATION

(45) Date of publication of patent specification :
**15.07.92 Bulletin 92/29**

(51) Int. Cl.⁵ : **A61F 13/50**

(21) Application number : **89912521.5**

(22) Date of filing : **16.11.89**

(86) International application number :
**PCT/SE89/00662**

(87) International publication number :
**WO 90/05514 31.05.90 Gazette 90/12**

(54) **A DISPOSABLE ABSORBENT ARTICLE.**

(30) Priority : **16.11.88 SE 8804136**

(43) Date of publication of application :
**04.09.91 Bulletin 91/36**

(45) Publication of the grant of the patent :
**15.07.92 Bulletin 92/29**

(84) Designated Contracting States :
**BE DE FR GB NL SE**

(56) References cited :
**GB-A- 2 100 130**
**US-A- 4 059 114**
**US-A- 4 650 481**

(73) Proprietor : **Mölnlycke AB**
**S-405 03 Göteborg (SE)**

(72) Inventor : **LINDQUIST, Bengt**
**Ryd Västergardsv. 30**
**S-443 51 Lerum (SE)**

(74) Representative : **Kierkegaard, Lars-Olov et al**
**H. ALBIHNS PATENTBYRA AB P.O. Box 3137**
**S-103 62 Stockholm (SE)**

Jouve, 18, rue Saint-Denis, 75001 PARIS

## Description

The present invention relates to a disposable absorbent article, according to the first part of claim 1. Such an article is disclosed in GB-A-2 100 130.

Leakage which occurs when using articles of this nature is not normally because the article has an insufficient total-absorption capacity, but because the liquid absorbed thereby is not dispersed effectively throughout the absorbent body. Consequently, the absorption capacity of the article is likely to be exceeded locally, with leakage as a result.

For the purpose of improving the dispersion of liquid in the longitudinal direction of the absorbent body of a sanitary towel, it has been proposed, e.g., in US Patent Specification No, 4,184,498 to provide a channel-forming impression in the absorbent pads or bodies forming part of such towels. This channel-forming impression is intended to guide liquid excretion centrally along the absorbent pad, and therewith to enable the total absorption capacity of the towel to be utilized more effectively. Thus, in order to achieve this improved dispersement of body liquid along the towel, it is necessary for the liquid excreted by the wearer to be deposited approximately in the centre of the absorbent pad.

In the case of sanitary towels, however, leakage is most often due to the fact that the towel has been positioned wrongly in the panties of the wearer, or has subsequently moved out of position, or has become deformed as a result of movement of the wearer's body. In such cases, the wetting point, by which is meant the location at which the body liquid is deposited on the absorbent body or pad, will lie to one side of the longitudinal symmetry-plane of the towel. This increases the risk that the local absorption-capacity of the pad will be insufficient to prevent leakage from occurring at the sides of the towel. This risk becomes greater with increasing distances of the wetting point from the centre of the absorbent body.

For the purpose of increasing the tolerance of sanitary towel to wrong positioning, there is proposed in European Patent Application EP 137 725 a sanitary towel in which the density of the absorbent pad increases slightly from the centre of the pad and laterally outwards thereof, and then increases drastically in a narrow region which extends around the full periphery of the pad. This pad-configuration is intended to prevent lateral leakage, by rapidly dispersing liquid which reaches the highly compressed peripheral region of the pad around the periphery of said pad.

In the case of this particular sanitary-napkin construction, however, the liquid is guided laterally by capillary action to the peripheral regions of the towel, and it is therefore these regions which are the first to become saturated with liquid excrement, and consequently, leakage as a result of local saturation is unavoidable. Furthermore, with a sanitary towel of this known construction, when the liquid has reached the highly compressed region of the absorbent pad, longitudinal dispersement of the liquid will take place solely in this region of the pad, and consequently liquid will leak from the sides of the towel before the absorption capacity of the less firmly compressed parts of the pad can be utilized.

The object of the present invention is to provide a disposable absorbent article which has good liquid-dispersion properties in both the longitudinal and transversal direction of the absorbent body and which also has good shape-stability, such that its ability to conform to the wearer's body will not be less than conventional articles of this kind, at least not to any appreciable extent.

This object is achieved in accordance with the invention by means of the features disclosed in the second part of claim 1. Because the channels formed by said impressions have transverse parts, the effectiveness of the article is not reliant on precisely where the wetting point is located on the absorbent body, and said channels enable the sanitary towel to function efficiently even when the position of the towel deviates from a correct towel position, for instance as a result of being wrongly positioned initially or of being subsequently disturbed.

Furthermore, the channel-forming impressions impart good stability to the article, in both its longitudinal and lateral directions, and consequently any disturbances in the position of the article when worn or any deformation to which the article is subjected will only be small.

These and other features of the invention, together with advantages afforded thereby, will be more readily understood from the following description of preferred embodiments of the invention and from the accompanying drawings, in which

Fig. 1 illustrates a first embodiment of an inventive sanitary towel;

Fig. 2 is a sectional view taken on the line II-II in Fig. 1;

Figs. 3A-3D illustrate examples of various combinations of semi-circular channels which can be pressed into a towel constructed in accordance with the invention;

Fig. 4 illustrates further variants of channel-forms which can be used in accordance with the invention; and

Fig. 5 illustrates a sanitary towel provided with esthetically attractive channels in accordance with the invention.

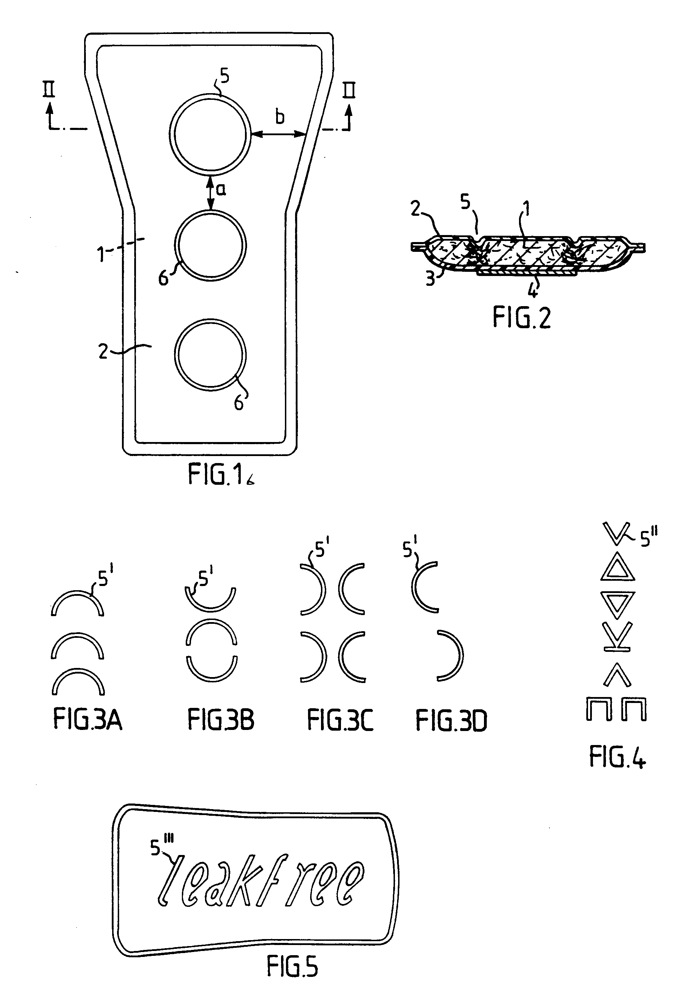

The sanitary towel illustrated in Figs. 1 and 2 includes an absorption pad 1 made of an absorbent material, for instance cellulose fluff, and two casing sheets 2 and 3, which enclose the absorption pad 1 and which are joined together along the parts thereof protruding beyond the pad, as illustrated in Fig. 2. The outer casing sheet 3 is impermeable to liquid, whereas the inner casing sheet 2, which is intended to lie against the wearer's body in use, consists of a liquid-permeable material. The sanitary towel also includes a double-sided adhesive tape 4 or the like attached to the outer casing sheet 3, so as to enable the towel to be secured firmly to the panties of the wearer.

The sanitary towel has pressed therein a longitudinally extending row of circular channels 5, 6 which are arranged symmetrically in relation to the longitudinal symmetry line of the towel and of which the channel 5 has a larger diameter than the channels 6.

The liquid-dispersion properties of the inventive sanitary towel will now be explained in respect of different wetting points.

If the towel is positioned so that the wetting point will be located within the circle 5, the liquid will disperse relatively slowly, in a radial direction relative to the wetting point, until it reaches the area located beneath the channel 5 on the absorbent pad, this underlying area having been compressed when making the channel-forming impression 5. Because the capillaries in this underlying area are smaller than the capillaries in the surrounding area, liquid will be transported more rapidly within this area and consequently liquid in the compressed area of the absorbent body beneath the channel 5 will be dispersed very rapidly. The transportation of liquid from the wetting point to the channel area will also be more rapid as a result of the capillary-suction effect obtained in a direction from larger to smaller capillaries. Thus, subsequent to liquid having reached the area of the channel 5, transportation of liquid continues essentially from the wetting point to the area which first reaches the channel area, and from there peripherally around the channel 5.

When or if the channel area becomes saturated, dispersion of the liquid continues radially from said channel area until the liquid reaches the compressed region of the absorption body located beneath the channel 6 adjacent the channel 5. The liquid is then dispersed rapidly in the channel area 6, until this area becomes saturated, whereafter the liquid is slowly dispersed to the next channel area, and so on.

In order to prevent lateral leakage of the liquid during this liquid-dispersement process, the distance a between the various channels shall be smaller than the distance b between the periphery of the channels and the nearest edge of the absorption body. If liquid is excreted to the wetting point subsequent to saturation of the channel area 6, the slow radial dispersion of liquid will take place more rapidly around the periphery of the channel 5 than around the periphery of the channel 6, and consequently lateral leakage should occur prior to the liquid reaching the next-following channel 6, unless the circular channels lie very close together.

If the towel is positioned so that the wetting point is located laterally outside the channel 5, but closer to the channel than to the edge of the absorption body, the liquid will be dispersed rapidly in the channel area as soon as it reaches this area. During the subsequent, slow dispersion of the liquid, the liquid will be dispersed more quickly around the wetting point than around the periphery of the channel area, and consequently there is a serious risk that lateral leakage will occur.

If the towel is positioned so that the wetting point is located between the various channels 5, 6, but within the cross-dimensions thereof, the liquid will be dispersed rapidly in that channel area which lies radially nearest the wetting point, subsequent to the liquid reaching said area. During the subsequent radial dispersion of liquid around the wetting point, the liquid will also reach adjacent channel areas, resulting a rapid dispersion of the liquid in said area.

Against this background, it will be understood that the cross-dimensions of the channels should be selected so that the wetting point will lie inwardly of the lateral borders of the channels and such that the distance between the channels will not be greater than the distance of the lateral borders of said channels from the nearest edge of the absorption body. It will also be understood that the dispersion of liquid along the length of the sanitary towel becomes more efficient the closer the channels are placed together, and hence the distance between the channels is selected so as to obtain the desired rigidity of the towel in its longitudinal direction.

As a result of the lateral extension of the channels, the channels provide a stiffening effect which imparts good lateral stability to the towel. Furthermore, the distances between the circular channels pressed in the towel can be selected so that the towel will conform to the configuration of those parts of the wearer's body against which the sanitary towel abuts when in use.

Many variations are possible within the scope of the invention. For instance, the sanitary towel may incorporate several rows of circular channels disposed along the length of the towel, and the channels formed may have a shape different to that shown. Figs. 3A-3D illustrate examples of channel patterns, in which semi-circular channels 5' are used. Fig. 4 illustrates other channel shapes 5", which can be used to produce longitudinally extending patterns. Fig. 5 illustrates an esthetically attractive patterns of channels 5''', which form a row of letters.

In the embodiment illustrated in Fig. 5, the channels 5''' lie very close together in the longitudinal direction of the towel, although all the channels have a relatively small extension in the vertical direction. Thus, the longitudinal flexibility of the towel illustrated in Fig. 5 is achieved by a large number of "hinge sections" formed between the mutually sequential channels in the longitudinal direction of the sanitary towel.

Although the invention is particularly suitable for application with sanitary towels, in which the excretion of liquid is relatively slow, it will be understood that the principles of the present invention can be utilized to advantage with other disposable absorbent articles, such as incontinence guards and diapers. Because an incontinence guard and a diaper will have larger dimensions than a sanitary towel, several rows of channels can be arranged in the longitudinal direction of the article, without the periphery of the channels being located too close to the edge of the absorbent body of the article concerned. Since the channels retain their shape, even when wet, the stability of the article is improved considerably, which is highly beneficial in the case of diapers in particular, which are exposed to a practically instantaneous excretion of a large quantity of liquid.

The invention thus provides a disposable absorbent article which has good dispersion properties in both the longitudinal and transversal directions thereof, and which also exhibits good lateral stability. Furthermore, the invention enables the stiffness properties of the article to be varied within relatively wide limits, while retaining good liquid-dispersion properties and while imparting an esthetically attractive pattern to the article at the same time.

## Claims

1. An absorbent article, such as a sanitary towel, an incontinence guard or a diaper, which includes an absorbent body (1) made of cellulose-fluff or some like absorbent material and which is enclosed between an outer liquid-impermeable sheet (3) and an inner liquid-permeable sheet (2), characterized in that the article includes a plurality of mutually sequential channel-forming impressions (5, 6; 5′, 5″, 5‴) unconnected to each other, which have both transverse and longitudinally extending parts and which are located at a distance from the periphery of the absorption body and separated from each other in the longitudinal direction of the article with a distance (a) between mutually adjacent channels (5, 6, 5′, 5″, 5‴) smaller than the distance (b) of respective channels to the nearest edge of the absorbent body (1).

2. An article according to Claim 1, characterized in that the channels (5, 6, 5′, 5″, 5‴) formed by said impressions are arranged symmetrically in relation to the longitudinal symmetry-plane of the article.

3. An article according to Claim 2, in which said article is a sanitary towel, characterized in that the transverse extent of each channel (5, 6, 5′, 5″, 5‴) is smaller than half the width of the sanitary towel at that location of the towel on which a respective channel is located.

4. An article according to any one of the preceding Claims, characterized in that the channels (5, 6) are circular in shape.

5. An article according to any one of Claims 1-3, characterized in that the channels (5′) are semi-circular in shape.

6. An article according to any one of Claim 1-3, characterized in that the channels (5″) are comprised of straight parts.

7. An article according to any one of Claims 1-3, characterized in that the channels (5‴) have the form of letters.

## Patentansprüche

1. Absorbierender Wegwerfartikel, wie z.B. eine Damenbinde, ein Inkontinenz-Schutz oder eine Windel, bestehend aus einem absorbierenden Kern aus Zellulose-Flocken o.dgl. absorbierendem Material, das zwischen einer äußeren, flüssigkeitsundurchlässigen Schicht (3) und einer inneren, flüssigkeitsdurchlässigen Schicht (2) eingeschlossen ist, dadurch gekennzeichnet, daß der Artikel mehrere, wechselseitig aufeinanderfolgende, kanalbildende und nicht miteinander verbundene Einpressungen (5, 6; 5′, 5″, 5‴) aufweist, die sowohl sich längs als auch quer erstreckende Teile haben und die in einem Abstand vom Rand des Absorptionskerns angeordnet sind und die ferner in Längsrichtung des Artikels mit einem Abstand (a) zwischen wechselseitig benachbarten Einpressungen (5, 6; 5′, 5″, 5‴) angeordnet sind, der kleiner ist als der Abstand (b) der jeweiligen Einpressungen von der nächsten Kante des absorbierenden Kernes (1).

2. Artikel nach Anspruch 1, dadurch gekennzeichnet, daß die kanalbildenden Einpressungen (5, 6; 5′, 5″, 5‴), die durch die Einpressungen gebildet worden sind, in bezug auf die Längs-Symmetrieebene des Artikels symmetrisch angeordnet sind.

3. Artikel nach Anspruch 2, bei dem der Artikel eine Damenbinde ist, dadurch gekennzeichnet, daß die Quer-Erstreckung jedes Kanals (5, 6; 5′, 5″, 5‴) an derjenigen Stelle der Damenbinde, an der der jeweilige Kanal liegt, kleiner ist als die halbe Breite der Damenbinde.

4. Artikel nach einem der vorangegangenen Ansprüche, dadurch gekennzeichnet, daß die Kanäle (5, 6) eine kreisrunde Form haben.

5. Artikel nach einem der Ansprüche 1 bis 3, dadurch gekennzeichnet, daß die Kanäle (5′) eine Halbkreisform haben.

6. Artikel nach einem der Ansprüche 1 bis 3, dadurch gekennzeichnet, daß die Kanäle (5″) aus geraden Teilen zusammengesetzt sind.

7. Artikel nach einem der Ansprüche 1 bis 3, dadurch gekennzeichnet, daß die Kanäle (5‴) die Form von Buchstaben haben.

## Revendications

1. Article absorbant, tel qu'une serviette hygiénique, une protection contre les incontinences ou une couche-culotte qui comprend un corps absorbant (1) formé d'un fluff de cellulose ou autre matière absorbante analogue et qui est enfermé entre une feuille extérieure (3) imperméable aux liquides et une feuille intérieure (2) perméable aux liquides, caractérisé en ce que l'article comprend une pluralité d'empreintes (5, 6; 5', 5'', 5''') de formation de canaux, non raccordées les unes aux autres, qui comportent des parties s'étendant à la fois transversalement et longitudinalement et qui se trouvent à une certaine distance de la périphérie du corps absorbant et sont séparées les unes des autres dans la direction longitudinale de l'article, la distance (a) entre les canaux mutuellement adjacents (5, 6, 5', 5'', 5''') étant plus petite que la distance (b) entre les canaux respectifs et le bord le plus près du corps absorbant (1).

2. Article selon la revendication 1, caractérisé en ce que les canaux (5, 6, 5', 5'', 5''') formés par lesdites empreintes sont disposés symétriquement par rapport au plan de symétrie longitudinal de l'artticle.

3. Article selon la revendication 2, dans lequel ledit article est une serviette hygiénique, caractérisé en ce que l'étendue transversale de chaque canal (5, 6, 5', 5'', 5''') est plus petite que la moitié de la largeur de la serviette hygiénique à l'endroit de la serviette où se trouve un canal correspondant.

4. Article selon l'une quelconque des revendications précédentes, caractérisé en ce que les canaux (5, 6) ont une forme circulaire.

5. Article selon l'une quelconque des revendications 1 à 3, caractérisé en ce que les canaux (5') ont une forme semi-circulaire.

6. Article selon l'une quelconque des revendications 1 à 3, caractérisé en ce que les canaux (5'') sont constitués de parties droites.

7. Article selon l'une quelconque des revendications 1 à 3, caractérisé en ce que les canaux (5''') ont la forme de lettres.

FIG.1

FIG.2

FIG.3A

FIG.3B

FIG.3C

FIG.3D

FIG.4

FIG.5